# EUROPÄISCHE PATENTANMELDUNG

(11) **EP 0 829 242 A1**
(43) Veröffentlichungstag der Anmeldung: **18.03.1998**
(21) Anmeldenummer: 96113229.7
(22) Anmeldetag: 19.08.1996
(51) Int. Cl.: A61F 2/06

(54) **Vorrichtung zum Entfernen einer implantierten Endoprothese**

(71) Anmelder: SCHNEIDER (EUROPE) AG, 8180 Bülach (CH)
(72) Erfinder: Gianotti Marc, CH-8542 Wiesendangen (CH)
(74) Vertreter: Althoff, Gerhard

(57) **Zusammenfassung**

Zum Entfernen einer bei einem Lebewesen in ein Hohlorgan implantierten und seibstexpandierend ausgebildeten Endoprothese (5) wird eine Vorrichtung vorgeschlagen, welche einen Transportkatheter (30) sowie eine darin in axialer Richtung verschiebbare Komprimiereinrichtung (50) umfasst.

Die einzelnen Elemente der Komprimiereinrichtung (50) sind so ausgebildet und zueinander angeordnet, dass bei einer in axialer Richtung orientierten Relativbewegung die von einer in radial aufgespreizter Eingriffposition gebrachte Greifeinrichtung (15) erfasste Endoprothese (5) etwa in Form einer sogenannten Hüllkurve (8) in radialer Richtung sukzessiv verjüngt in den als Fangrohr ausgebildeten Transportkatheter (30) gezogen werden kann. Die am distalen Ende eines Zuggliedes (10) angeordnete Greifeinrichtung (15) kann wiederholt mit der implantierten Endoprothese (5) in Eingriff und zwischen den Eingriffen vollständig von dieser gelöst werden.

## Beschreibung

Die Erfindung bezieht sich auf eine Vorrichtung zum Entfernen einer in ein Hohlorgan eines Lebewesens implantierten Endoprothese, insbesondere einer seibstexpandierend ausgebildeten Endoprothese, mit einem in das Hohlorgan einführbaren Transportkatheter und einer darin axial verschiebbar angeordneten Komprimiereinrichtung, mittels welcher die Endoprothese radial verjüngbar ist und in weitgehend komprimiertem Zustand in den Transportkatheter einziehbar sowie zusammen mit diesem aus dem Hohlorgan entfernbar ist.

Zur Korrektur der Lage einer in ein Blutgefäss oder dergleichen implantierten Endoprothese ist aus der EP-A 0 575 719 eine Vorrichtung bekannt, bei welcher in einem Einführschlauch ein in Form eines länglichen Drahtteils ausgebildetes Zugglied angeordnet ist, welches an dem distalen Ende etwa zu einem Körbchen radial aufstellbare Drahtabschnitte aufweist, die mittels daran angeordneter Haken zum Ziehen (proximal) mit der Endoprothese in Eingriff bringbar ist. Bei dieser Vorrichtung sind keine Mittel zum radialen Komprimieren der Endoprothese und auch kein Transportkatheter zur Aufnahme und zum Entfernen derselben sowie zum Schutz der Gefässinnenwand vorgesehen. Die Vorrichtung ist ausschliesslich für die Durchführung einer geringfügigen Lagekorrektur der Endoprothese innerhalb des Blutgefässes ausgebildet.

Aus der US-A 5,098,440 ist eine Vorrichtung zum Positionieren und/oder Entfernen einer in ein Blutgefäss oder dergleichen implantierten Endoprothese (Stent) bekannt, welche einen Transportkatheter, einen koaxial darin angeordneten Innenkatheter sowie zwei darin getrennt voneinander in axialer Richtung verschiebbar angeordnete Zugglieder umfasst, welche am distalen Ende jeweils mit einem als Schlinge ausgebildeten Greiforgan versehen sind. Bei dieser Vorrichtung besteht der Nachteil, dass die zu entfernende Endoprothese mittels der Schlingen lediglich lokal eingeschnürt und somit in weitgehend expandiertem Zustand entlang der leicht verletzbaren Gefässinnenwand in den entsprechend gross zu dimensionierenden und folglich nicht bis unmittelbar an die Endoprothese heranführbaren Transportkatheter gezogen wird. Der Transportkatheter erfordert zudem eine relativ grosse Punktion zum Einführen desselben, so dass der Anwendungsbereich dieser Vorrichtung verhältnismässig stark begrenzt ist.

Zum Entfernen einer in ein Hohlorgan eines Lebewesens implantierten und selbstexpandierend ausgebildeten Endoprothese ist aus der EP-A 0 423 916 eine Vorrichtung bekannt, welche einen Transportkatheter, einen koaxial darin angeordneten und in axialer Richtung relativ dazu verschiebbaren Innenkatheter aufweist, in welchem mindestestens zwei in axialer Richtung orientierte und ständig mit der Endoprothese wirkverbundene sowie in Abhängigkeit der Anwendung mit unterschiedlicher Länge ausgebildete Zugglieder in Form von Fäden oder dergleichen angeordnet sind, mittels welcher durch proximales Ziehen die beispielsweise aus Drähten in Zickzack-Anordnung ausgebildete Endoprothese etwa durch Zusammenfalten radial verjüngend komprimiert und zum Entfernen zusammen mit dem Innenkatheter in den Transportkatheter gezogen sowie mit diesem aus dem Hohlorgan entfernt wird. Bei dieser Vorrichtung ist es erforderlich, dass die einzelnen Zugglieder sowohl beim Implantieren und Expandieren der Endoprothese als auch während des gesamten Eingriffs permanent mit derselben verbunden sind, wodurch der Anwendungsbereich der Endoprothese relativ stark begrenzt und zudem nur für temporäre Einsätze geeignet ist.

Der Erfindung liegt die Aufgabe zugrunde, eine Vorrichtung gemäss der im Oberbegriff des Anspruchs 1 genannten Gattung dahingehend zu verbessern, dass insbesondere die während des Eingriffs als störend empfundene permanente Verbindung der Zugglieder mit der implantierten Endoprothese nicht erforderlich ist und die Endoprothese mit relativ einfachen Mitteln ohne die Gefässinnenwand zu verletzen aus dem Hohlkörper entfernbar ist.

Diese Aufgabe wird gemäss der Erfindung dadurch gelöst, dass die Komprimiereinrichtung zum Entfernen einer bereits implantierten Endoprothese wiederholt mit dieser in Eingriff bringbar und zwischen den Eingriffen vollständig von dieser lösbar ist.

Hierdurch ist es möglich eine in ein Hohlorgan oder dergleichen implantierte Endoprothese, insbesondere eine sich bis zur formschlüssigen Anlage an die Innenwand des Hohlorgans seibstexpandierend erweiternde Endoprothese, vor dein Herausziehen durch die Relativbewegungen der einzelnen Funktionselemente in vorteilhafter Weise von der Innenwand des Hohlorgans sukzessive zu lösen sowie radial zu verkleinern und gleichzeitig in den Transportkatheter einzuziehen, welche anschliessend zusammen mit Zugglied und Innenkatheter aus dem Hohlorgan entfernbar ist.

Der konstruktive Aufbau der Komprimiereinrichtung gewährleistet eine verhältnismässig einfache Handhabung während des Eingriffs und ein sicheres Erfassen der zu entfernenden Endoprothese. Im Rahmen der Erfindung hat sich herausgestellt, dass die in das Hohlorgan einzuführenden Elemente (Transportkatheter, Innenkatheter und Zugglied) auch bei längeren Abständen von der Punktionsöffnung vorzugsweise aus biegsamem Material, beispielsweise aus geeignetem Kunststoff hergestellt werden können, so dass sich diese beim Einführen an die innere Formgebung des Hohlorgans entsprechend anpassen können ohne dabei die Innenwand zu verletzen. Bei kürzeren Distanzen von der Punktionsöffnung können die einzelnen Elemente auch aus relativ steifem Material hergestellt werden.

Weitere Merkmale der Erfindung ergeben sich aus der folgenden Beschreibung in Verbindung mit dem in der Zeichnung dargestellten Ausführungsbeispiel und den Patentansprüchen.

Die Erfindung wird nachstehend anhand der Zeichnung beschrieben. Es zeigt:
**Fig.1** das distale Ende einer in grösserem Massstab sowie in Schnittansicht dargestellten erfindungsgemässen Vorrichtung mit einer in Verschlussposition dargestellten Greifeinrichtung;
**Fig.2** das distale Ende der Vorrichtung gemäss Fig.1 mit der in radial gespreizter Eingriffsposition dargestellten Greifeinrichtung;
**Fig.3** die gemäss Pfeilrichtung X in Fig.2 in Ansicht dargestellte Greifeinrichtung mit den einzelnen radial nach aussen gespreizt dargestellten Greifelementen;
**Fig.4** ein teilweise und in grösserem Massstab dargestelltes Teilstück eines einzelnen Greifelements für die Greifeinrichtung;
**Fig.5** die teilweise in das proximale Ende einer Endoprothese eingeführte Komprimiereinrichtung gemäss Fig.1 mit der in Verschlussposition dargestellten Greifeinrichtung;
**Fig.6** die weiter in axialer Richtung in das proximale Ende der Endoprothese eingeführte Komprimiereinrichtung mit der in radial aufgespreizter Eingriffsposition dargestellten Greifeinrichtung;
**Fig.7** die in dem proximalen Ende der Endoprothese angeordnete Komprimiereinrichtung gemäss Fig.6 mit den an Filamenten eingehakt dargestellten Greifelementen der Greifeinrichtung; und
**Fig.8** das mittels der eingehakten Greifelemente in Form einer theoretischen Hüllkurve radial verjüngte und teilweise in einen Transportkatheter eingezogene proximale Ende der Endoprothese.

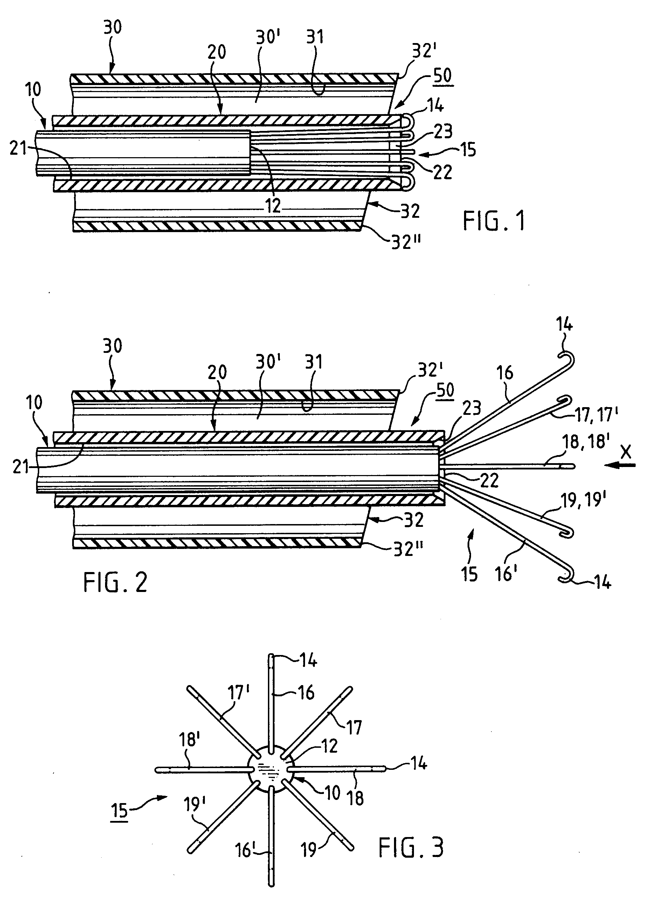

Fig.1 zeigt zur Verdeutlichung der Erfindung ein in Schnittansicht dargestelltes distales Ende einer Komprimiereinrichtung, welche etwa in Form eines länglichen chirurgischen Instruments zum Einführen in ein Hohlorgan, beispielsweise zum Einführen in ein Blutgefäss eines Lebewesens ausgebildet ist. Im dargestellten Ausführungsbeispiel ist die Komprimiereinrichtung insbesondere zum Entfernen einer in ein Hohlorgan implantierten selbstexpandierenden Endoprothese (Stent) ausgebildet, ohne dass beim Entfernen (Extraktion) die Endoprothese an der Innenwand hängen bleibt und dadurch unerwünschte Verletzungen entstehen können. Die erfindungsgemässe Komprimiereinrichtung kann jedoch auch unter Beibehaltung der vorstehenden Bedingungen ohne zusätzliche Hilfsmittel zum proximalen Nachpositionieren der in ein Hohlorgan oder dergleichen eingesetzten Endoprothese verwendet werden.

Die in der Gesamtheit mit 50 bezeichnete und in grösserem Massstab dargestellte Komprimiereinrichtung umfasst im wesentlichen ein Zugglied 10 mit distalseitig angeordneter Greifeinrichtung 15 und einen Innenkatheter 20. In zusammengebautem Zustand ist das Zugglied 10 etwa koaxial in dem Innenkatheter 20 und dieser zusammen mit dem Zugglied 10 etwa koaxial in einem Transportkatheter 30 angeordnet. Die mit einer nicht näher dargestellten Betätigungseinrichtung wirkverbundenen und in axialer Richtung relativ zueinander verschiebbaren Elemente 10, 15, 20 und 30 werden nachstehend in Verbindung mit den Figuren 1 bis 4 im einzelnen beschrieben.

Das Zugglied 10 ist an seinem proximalen Ende für die in axialer Richtung orientierte Bewegung beispielsweise mit einem nicht dargestellten Griffteil (Betätigungsvorrichtung) versehen. An dem distalen Ende ist an der Stirnseite 12 die in Fig.1 in der Verschlussposition dargestellte Greifeinrichtung 15 angeordnet. Die Greifeinrichtung 15 umfasst mindestens zwei, vorzugsweise aber mehrere an der Stirnseite 12 gleichmässig verteilt angeordnete und in nicht dargestellter Weise befestigte Greifelemente 16,16';17,17';18,18' und 19, 19' (Fig.2). Das Zugglied 10 kann auch aus einem hohlzylindrischen Rohr hergestellt werden. Bei dieser nicht dargestellten Variante ist am distalen Ende des Zuggliedes 10 eine Platte zur Befestigung der Greifeinrichtung 15 angeordnet und befestigt.

Der hohlzylindrische Innenkatheter 20 ist an der distalen Stirnseite 22 mit einer abgeschrägt oder abgerundet ausgebildeten, zirkulären Anlagekante 23 versehen. Die von der äusseren Stirnseite 22 nach innen abgeschrägt oder abgerundete Anlagekante 23 gewährleistet beim Öffnen der Greifeinrichtung 15, dass die einzelnen Greifelemente 16,16';17,17';18,18' und 19,19' entlang der Anlagekante 23 gleiten und durch die radial nach aussen gerichtete Federkraft gleichmässig radial nach aussen gespreizt werden (Fig.2). Beim Einziehen gleiten die einzelnen Greifelemente 16,16';17,17';18,18';19,19' entlang der Anlagekante 23 und werden dabei entgegen der Federkraft gleichmässig radial zusammengeklappt in den Innenkatheter 20 gezogen. In eingezogenem Zustand sind die einzelnen Greifelemente 16,16';17,17';18,18' und 19,19' etwa im distalen Bereich der Innenwand 21 des Innenkatheters 20 fest anliegend angeordnet und die angeformten Haken 14 (nur einmal bezeichnet) sind in der Verschlussposition (Fig.1) gegen die kreisringförmige Stirnseite 22 des Innenkatheters 20 gezogen.

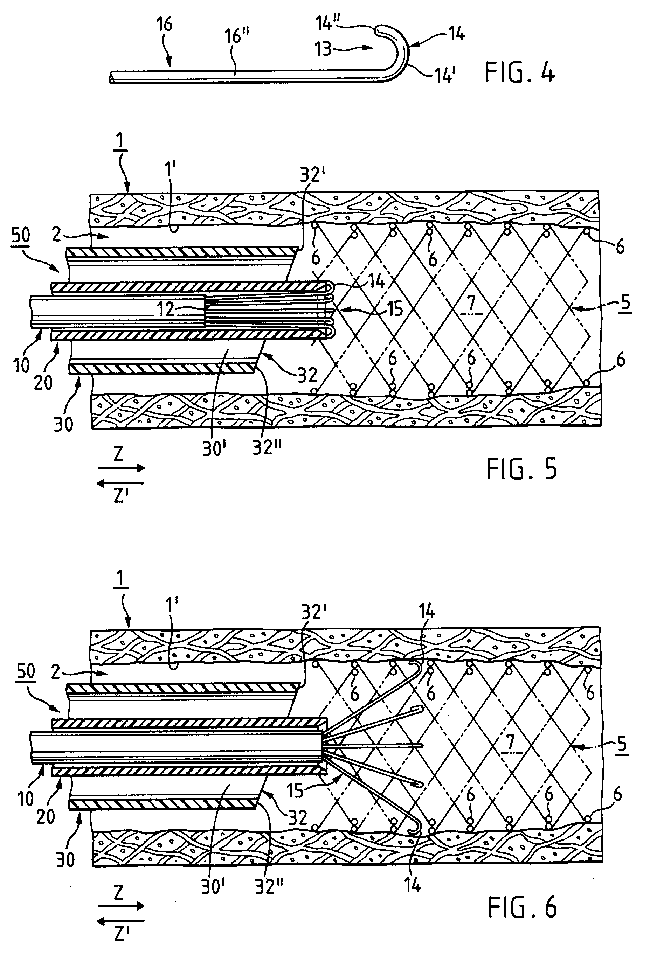

Der hohlzylindrische Transportkatheter 30 ist am distalen Ende zum besseren Einführen der zu entfernenden Endoprothese mit einer abgeschrägten Stirnfläche 32 versehen. Die Abschrägung der Stirnfläche 32 ist ausgehend von der oberen, beim Einführen in den Innenraum 2 des Hohlorgans 1 der Endoprothese 5 (Fig.5 bis 8) näher liegenden Stirnkante 32' in Richtung der unteren Stirnkante 32'' rückwärts geneigt ausgebildet. Die Abschrägung der kreisringförmigen Stirnfläche 32 ist vorzugsweise unter spitzem Winkel in der Grössenordnung von 10° bis 25° ausgebildet.

Fig.2 zeigt die Komprimiereinrichtung 50, bei welcher der Innenkatheter 20 in axialer Richtung relativ zu dem Transportkatheter 30 und das Zugglied 10 in axialer Richtung relativ zu dem Innenkatheter 20 verschoben dargestellt sind. Beim Verschieben des Zuggliedes 10 relativ zu dem Innenkatheter 20 gleiten die einzelnen Greifelemente 16,16';17,17';18,18' und 19,19' durch die vorgegebene Federkraft entlang der abgeschrägten Anlagekante 23 des Innenkatheters 20 bis die maximale Eingriffposition erreicht ist. In dieser Position sind die an der Stirnseite des Zuggliedes 10 angeordneten Greifelemente 16,16';17,17';18,18' und 19,19' nicht mehr gegen die abgeschrägte Kante 23 des Innenkatheters 20 gespannt.

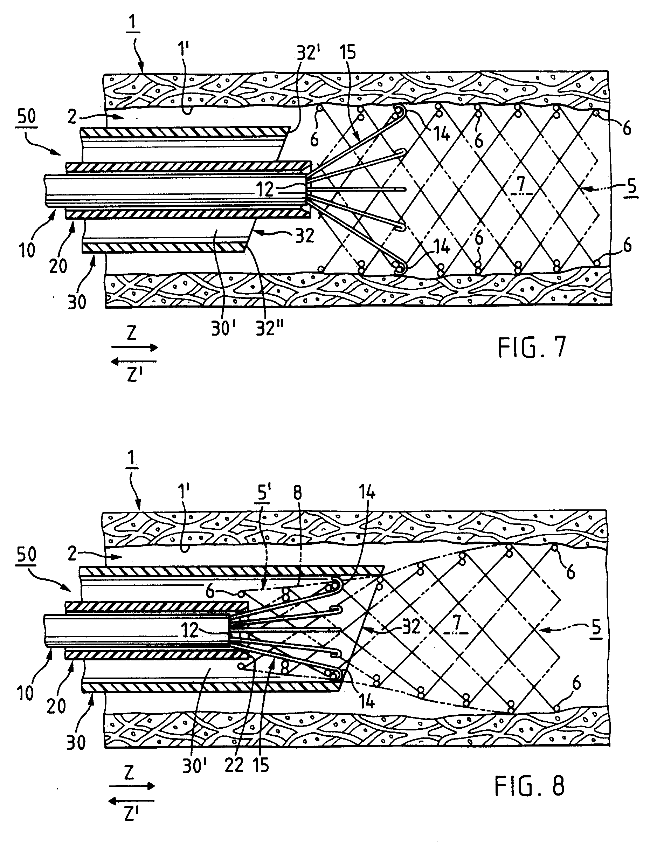

Wie in Fig.1 und Fig.2 dargestellt, ist der Innenkatheter 20 vorzugsweise koaxial in dem Transportkatheter 30 angeordnet, so dass zwischen der Innenwand 31 des Transportkatheters 30 und dem Innenkatheter 20 ein weitgehend hohlzylindrischer Innenraum 30' entsteht. In den Innenraum 30' kann beim Einziehen der aus dem Hohlorgan 1 zu entfernenden Endoprothese 5 in den Transportkatheter 30 das proximale Ende der Endoprothese 5 hineinragen (Fig.8).

In Fig.3 ist gemäss Pfeilrichtung X in Fig.2 die an der Stirnseite 12 des Zuggliedes 10 angeordnete Greifeinrichtung 15 dargestellt und man erkennt die einzelnen in Umfangsrichtung verteilt angeordneten und an den äusseren Enden jeweils mit einem Haken 14 versehenen Greifelemente 16,16';17,17'; 18,18' und 19,19', welche in bezug auf die nicht näher dargestellte Längsachse des Zuggliedes 10 schräg nach aussen gerichtet an der Stirnseite 12 desselben angeordnet und mit nicht dargestellten Mitteln befestigt sind.

In Fig.4 ist als Ausführungsbeispiel das als Haken 14 ausgebildete Teilstück des Greifelements 16 in grösserem Massstab dargestellt. Der angeformte Haken 14 ist in bezug auf das gerade Teilstück 16'' des stabförmigen Greifelements 16 mit einem umgebogenen Bogenteil 14' versehen, welches einen Winkel von mindestens 90°, vorzugsweise aber einen Winkel in der Grössenordnung von 120° bis 180° aufweist. Das freie Ende 14'' des Bogenteils 14' ist zur Vermeidung von Verletzungen in nicht näher dargestellter Weise abgerundet ausgebildet. Der zwischen dem geraden Teilstück 16'' und dem Bogenteil 14' vorgesehene Abstand 13 ist derart bemessen, dass ein sicheres Erfassen und Halten des einzelnen Filaments 6 der Endoprothese 5 (Fig.7) sowie ein Lösen der eingehängten Greifelemente von den Filamenten 6 gewährleistet ist. Die an den einzelnen Greifelementen 16,16';17,17';18,18' und 19,19' angeformten und nicht näher bezeichneten Haken sind analog dem vorstehend beschriebenen Haken 14 ausgebildet.

Die Funktionsweise der erfindungsgemässen Vorrichtung wird nachstehend in Verbindung mit den Figuren 5 bis 8 im einzelnen beschrieben:

In Fig.5 ist die erste Phase dargestellt, bei welcher die Komprimiereinrichtung 50 zusammen mit dem Transportkatheter 30 mit nicht dargestellten Mitteln in den Innenraum 2 des Hohlorgans 1 eingeführt und bis etwa in den vorderen Bereich der implantierten Endoprothese 5 geschoben ist. In dieser Phase ist die Greifvorrichtung 15 noch in radial zusammengeklapptem Zustand in dem gemäss Pfeilrichtung Z relativ zu dem Transportkatheter 30 teilweise in den Innenraum 7 der Endoprothese 5 geschobenen Innenkatheter 20 angeordnet.

In einer zweiten Phase ist, wie in Fig.6 schematisch dargestellt, das Zugglied 10 gemäss Pfeilrichtung Z relativ zu dem Innenkatheter 20 verschoben. Die dabei von dem Innenkatheter 20 sukzessive freigegebenen Greifelemente 16,16';17,17';18, 18' und 19,19 werden durch die Federkraft radial nach aussen gespreizt gegen die nicht dargestellte Innenwand der Endoprothese 5 gedrückt, derart, dass die einzelnen Greifelemente 16,16';17,17';18,18' und 19,19 mit den daran angeformten Haken 14 zwischen zwei axial beabstandete Filamente 6 der Endoprothese 5 eingreifen (Fig.6).

Wie in Fig.7 schematisch dargestellt, wird zum einhängenden Erfassen der den einzelnen Greifelementen 16,16';17,17';18, 18' und 19,19 zugeordneten Filamente 6 das Zugglied 10 gemäss Pfeilrichtung Z' in den Innenkatheter 20 gezogen. Hierbei werden die vorderen von den einzelnen Greifelementen 16,16'; 17,17';18,18' und 19,19 erfassten Filamente 6 in nicht näher dargestellter Weise von der Innenwand 1' des Hohlorgans 1 gelöst.

In der in Fig.8 dargestellten Phase wird das vordere von den einzelnen Greifelementen 16,16';17,17';18,18' und 19,19 erfasste Ende 5' der Endoprothese 5 in Form einer sukzessive radial verjüngten und durch eine theoretische Linie 8 dargestellte Hüllkurve entlang der abgeschrägten Stirnseite 32 in den Transportkatheter 30 gezogen. Hierbei besteht die Möglichkeit, dass der Transportkatheter 30 mit der distalen Stirnseite 32 in Richtung der Endoprothese 5 relativ zu dem Innenkatheter 20 und dem Zugglied 10 in Pfeilrichtung Z vorgeschoben oder der Innenkatheter 20 mit dem Zugglied 10 relativ zu dem Transportkatheter 30 in Pfeilrichtung Z' zurückgezogen wird. Sobald die Endoprothese 5 mit ihrer gesamten Länge in den Transportkatheter 30 gezogen ist, kann die Komprimiereinrichtung 50 mit der eingezogenen Endoprothese 5 aus dem Hohlorgan 1 entfernt werden.

Der konstruktive Aufbau der gesamten Komprimiereinrichtung 50 gewährleistet eine verhältnismässig einfache Handhabung und Manipulation während des Eingriffs sowie ein sicheres Erfassen und Entfernen (Extraktion) der Endoprothese 5. Das in Pfeilrichtung Z orientierte Vorschieben des als sogenanntes Fangrohr ausgebildeten Transportkatheters 30 bewirkt im vorderen Bereich ein Lösen der Endoprothese 5 von der Innenwand 1' des Hohlorgans 1 und im hinteren Bereich derselben eine Entlastung der etwa radial nach aussen wirkenden Spannkraft gegen die Innenwand 1'. Die mit den einzelnen Greifelementen versehene Greifeinrichtung 15 kann mit der implantierten Endoprothese 5 ohne zusätzliche Hilfsmittel wiederholt in Eingriff gebracht und zwischen den Eingriffen vollständig von der Endoprothese 5 gelöst werden.

Das Zugglied 10 und die beiden Katheter 20 und 30 sind beispielsweise aus geeignetem Kunststoff beziehungsweise aus einem mit einem Geflecht verstärkten Kunststoff hergestellt, so dass diese in bezug auf ihre Längsachse biegsam sind und problemlos anpassend in den Innenraum 2 des Hohlorgans 1 eines Lebewesens zum Erfassen einer implantierten Endoprothese 5 eingeführt werden können.

Die Endoprothese 5 besteht beispielsweise aus gegenläufig miteinander verflochtenen Gruppen von schraubenlinienförmig gewundenen Filamenten 6 aus Draht oder dergleichen und ist derart ausgebildet, dass diese durch eine in axialer Richtung daran wirkende geringe Zugkraft sich selbsttätig radial verkleinert und somit problemlos in den Innenraum des als Fangrohr ausgebildeten Transportkatheters 30 gezogen werden kann. Die Endoprothese 5 ist nicht Gegenstand dieser Erfindung und deshalb in den Figuren 5 bis 8 nur schematisch dargestellt und nicht näher beschrieben.

## Patentansprüche

1. Vorrichtung zum Entfernen einer in ein Hohlorgan eines Lebewesens implantierten Endoprothese, insbesondere einer seibstexpandierend ausgebildeten Endoprothese, mit einem in das Hohlorgan einführbaren Transportkatheter und einer darin axial verschiebbar angeordneten Komprimiereinrichtung, mittels welcher die Endoprothese radial verjüngbar ist und in weitgehend komprimiertem Zustand in den Transportkatheter einziehbar sowie zusammen mit diesem aus dem Hohlorgan entfernbar ist, **dadurch gekennzeichnet**, dass die Komprimiereinrichtung (50) zum Entfernen einer bereits implantierten Endoprothese (5) wiederholt mit dieser in Eingriff bringbar und zwischen den Eingriffen vollständig von dieser lösbar ist.

2. Vorrichtung nach Anspruch 1, **dadurch gekennzeichnet**, dass die Komprimiereinrichtung (50) einen in dem Transportkatheter (30) angeordneten Innenkatheter (20), ein darin angeordnetes Zugglied (10) sowie eine an dem distalen Ende (12) des Zuggliedes (10) angeordnete und von dem Innenkatheter (20) in zusammengeklappter Verschlussposition gehaltene Greifeinrichtung (15) aufweist, welche infolge einer axialen Schiebebewegung des Zuggliedes (10) relativ zu dem Innenkatheter (20) oder umgekehrt in eine radial aufgespreizte Eingriffposition bringbar ist.

3. Vorrichtung nach Anspruch 2, **dadurch gekennzeichnet**, dass die Greifeinrichtung (15) mehrere sich durch eine radial nach aussen gerichtete Federkraft gegen die Innenwand (21) des Innenkatheters (20) spannende und jeweils mit einem Haken (14) versehene Greifelemente (16,16';17,17';18,18';19,19') aufweist, welche durch axiale Verschiebung des Zuggliedes (10) relativ zum Innenkatheter (20) aus der Verschlussposition in die Eingriffposition aufspreizbar und durch Einhängen der Haken (14) in die Endoprothese (5) mit dieser in Eingriff bringbar sind.

4. Vorrichtung nach den Ansprüchen 2 und 3, **dadurch gekennzeichnet**, dass das distale Ende des Innenkatheters (20) zur gleitenden Anlage und Führung der Greifelemente (16,16'; 17,17';18,18';19,19') innenseitig mit einer abgeschrägten oder abgerundeten Anlagekante (23) versehen ist.

5. Vorrichtung nach Anspruch 3, **dadurch gekennzeichnet**, dass der an dem einzelnen Greifelement (16,16';17,17';18, 18';19,19') angeformte Haken (14) mit einem Bogenteil (14') versehen ist, welches mindestens einen Winkel von 90°, vorzugsweise einen Winkel in der Grössenordnung zwischen 120° und 180° aufweist.

6. Vorrichtung nach Anspruch 3, **dadurch gekennzeichnet**, dass der an dem einzelnen Greifelement (16,16';17,17';18, 18';19,19') angeformte Haken (14) am Ende (14'') des Bogenteils (14') abgerundet ausgebildet ist.

7. Vorrichtung nach Anspruch 1, **dadurch gekennzeichnet**, dass der Transportkatheter (30) am distalen Ende abgeschrägt ist.
